# EUROPEAN PATENT APPLICATION

(11) **EP 1 818 053 A1**
(43) Date of publication of application: **15.08.2007**
(21) Application number: 05800538.0
(22) Date of filing: 01.11.2005
(51) Int. Cl.: A61K 31/198, A61K 45/00, A61P 11/06, A61P 37/08

(54) **PREVENTIVE/REMEDY FOR ALLERGIC DISEASES**

(30) Priority: 02.11.2004 JP 2004319640
(71) Applicant: Ajinomoto Co., Inc., Tokyo 104-8315 (JP)
(72) Inventor: YONEDA, Junya, Ajinomoto Co., Inc., Kawasaki-shi, Kanagawa 2108681 (JP); SAKAI, Ryosei, Ajinomoto Co., Inc., Kawasaki-shi, Kanagawa 2108681 (JP)
(74) Representative: Nicholls, Kathryn Margaret
(86) International application number: PCT/JP2005/020396
(87) International publication number: WO 2006/049286

(57) **Abstract**

Provided is a prophylactic/therapeutic agent for an allergic disease caused by Th2 cell-polarized activation, which comprises at least one kind selected from a branched amino acid, glycine, serine, arginine, a keto acid thereof and a salt thereof. Because the prophylactic/therapeutic agent of the present invention produces almost no side effects, it can be safely ingested routinely and can be taken for a long time. When the prophylactic/therapeutic agent of the present invention is used in combination with an anti-inflammatory drug, the dose of the anti-inflammatory drug can be reduced. Hence, the prophylactic/therapeutic agent of the present invention is useful for the prevention or treatment of an allergic disease caused by Th2 cell-polarized activation. The prophylactic/therapeutic agent of the present invention is particularly effective on bronchial asthma and pulmonary fibrosis that accompanies bronchial asthma.

## Description

### Technical Field

The present invention has been developed on the basis of the finding that at least one kind of amino acid (may be in any free form, a keto acid that can be converted to the free form in a living organism, or a salt thereof) selected from a branched amino acid, glycine, serine and arginine exhibits actions such as prevention of attacks, progression prevention, and amelioration in an allergic disease caused by Th2 cell-polarized activation, for example, an allergic disease represented by adult or child bronchial asthma, and a prophylactic/therapeutic action on pulmonary fibrosis that accompanies bronchial asthma, and provides pharmaceuticals (including infusions, nutritive drugs and the like) and foods (including medical foods, health foods, foods for specified health uses and the like). Furthermore, the present invention also relates to a prophylactic/therapeutic method for the above-described allergic disease, a use of the above-described amino acid in a prophylactic/therapeutic agent for an allergic disease, and the like.

### Background Art

Regarding the definition of bronchial asthma, according to the "Guideline for Prevention and Management of Asthma, 1988 Revised Edition", issued in 1988 by the Japanese Society of Allergology, "bronchial asthma is characterized by airway inflammation and various degrees of airflow restriction and causes paroxysmal coughing, wheezing, and dyspnea. The airflow restriction occurs in a range of severity, from mild to fatally severe, and is at least partially reversible by nature or by treatment. The airway inflammation is involved by infiltration of many inflammatory cells such as eosinophils, T cells (Th2), and mast cells, and airway mucosal epithelial damage is observed". The definition of child asthma is given in the "Guideline for Treatment of Allergic Diseases", also published by the Japanese Society of Allergology: "child bronchial asthma is a disease in which dyspnea paroxysmally accompanied by whistling wheezing occurs repeatedly, the resulting dyspnea being remitted and healed by nature or by treatment. Its pathologic profile consists of reversible anastomotic lesions and persistent inflammation involving the mucosa and muscular layer of the airway".

Bronchial asthma, like allergic rhinitis and atopic dermatitis, is a disease involved by allergic reactions (an allergic disease). An allergic reaction is an immune reaction that affects a living organism as a result of an excess reaction of Immune cells to an antigen that is harmless to a living organism. Usually, an allergen entering a living organism undergoes phagocytosis by antigen-presenting cells such as macrophages, dendritic cells, and B cells, and then antigen-presented to T cells in the form of a peptide by the action of the HLA class II antigen of self. The T cells are activated via T cell receptors and this activation is concurrently promoted by IL-1, IL-6 and the like secreted from the antigen-presenting cells. The CD4-positive helper T cells involved in the induction of immune responsive reactions are classified according to cytokine production pattern into two types: Th1 cells, which produce IFN-γ, IL-2 and the like, and Th2 cells, which produce IL-4, IL-5, IL-10 and the like. Th1 cells produce IFN-γ and activate macrophages to enhance the bactericidal potential against intracellular parasitic bacteria, and induce the differentiation and proliferation of CD8-positive cells by producing IL-2.

On the other hand, by the action of cytokines secreted from Th2 cells, inflammatory cells such as mast cells/basophils and eosinophils differentiate and proliferate, and the system for IgE production from B cells is promoted. Th1 cells and Th2 cells mutually regulate their functions; as Th1 cells are predominant, Th2 cell reactions are suppressed, and conversely as Th2 cells are predominant, Th1 cell reactions are suppressed. Thus, a living organism achieves the maintenance of homeostasis by balancing between the functions of Th1 and Th2 cells. An allergic disease is considered to be a state wherein Th2 cell reactions are extremely predominant over Th1 cell responses. The differentiation induction of helper T cells to Th1 cells or Th2 cells depends on the kind of antigen, the kind of antigen-presenting cells, and the cytokine in action. Allergens and parasitic antigens that induce IgE production are likely to induce Th2 cells, whereas viral and bacterial antigens are likely to induce Th1 cells. Of the cells that present these antigens to T cells, B cells are involved mainly in the activation of Th2 cells, whereas macrophages are involved mainly in the activation of Th1 cells. Of the dendritic cells, CD8-positive DC1 cells are involved in the activation of Th1 cells, whereas CD8-negative DC2 cells are involved in the activation of Th2 cells. Allergic diseases are Th2 diseases, which are characterized in that the function of Th2 cells is abnormally accentuated compared to that of Th1 cells, and can be regarded as diseases of a totally different mechanism from that of Th1 diseases, such as rheumatism, which are also inflammatory diseases but characterized by accentuation of the function of Th1 cells.

As pathophysiological features of bronchial asthma, (i) airway mucosal inflammation (infiltration of eosinophils, T cells, and mast cells, and airway epithelial damage), (ii) accentuation of airway reactivity considered to be due to the same, (iii) reversible airway stenosis of variously changeable degrees, (iv) persistent airway stenosis due to airway organic changes (remodeling), and the like can be mentioned. In patients with remarkable eosinophil infiltration in the airway, airway epithelial damage is remarkable and airway reactivity accentuation is observed; in patients with remarkable airway reactivity, asthma symptoms are severe. The degree of eosinophil infiltration is proportional to the degree of infiltration of type II CD4 + helper T cells (Th2 cells); cytokines produced by T cells, such as IL-5, are considered to enhance eosinophil infiltration. In asthma attacks, a variety of stimuli on the hypersensitive airway causes airway obstruction; regarding the mechanisms for the airway constriction, (i) constriction of bronchial smooth muscle, (ii) airway mucosal edema due to vascular permeability accentuation, (iii) mucosal retention in the airway due to accentuation of secretion in the airway, (iv) secretory excretion delay and airway constrictiveness accentuation due to airway cilated epithelial destruction, and the like are likely. The pathologic profile of child asthma is basically indifferent from that of adult bronchial asthma.

As symptoms of bronchial asthma, (i) symptoms due to airway obstruction, such as dyspnea, (ii) symptoms resulting directly from airway inflammation, such as coughing/sputum, and the like can be mentioned. These symptoms are characterized by fluctuation. Hence, the same asthma patient exhibits short breaths, wheezing, dyspnea, obstructive ventilation disorder, hypoxemic symptoms and the like due to severe airway obstruction during exacerbation, even with gait being hampered due to dyspnea in the event of an attack; however, during remission, their respiratory function and daily activities show almost no differences from those of healthy people. The same applies to airway reactivity; a slight stimulus easily causes asthma symptoms during exacerbation, but during remission, even the same stimulus does not induce symptoms.

In the above-described "Guideline for Prevention and Management of Asthma", cases of asthma are classified from the viewpoint of severity of asthma (intensity of symptoms) according to the intensity and frequency of attacks into four types to provide criteria for asthma treatment. Specifically, the Guideline classifies cases of asthma into (i) mild intermittent type: fully normal activities are possible during the attack-free period but coughing, wheezing, or dyspnea develops twice or less in a week, with a nocturnal attack occurring twice or less in a month; (ii) mild persistent type: an attack occurs twice or more in a week and daily activities or sleep is impaired, with a nocturnal attack occurring twice or more in a month; (iii) moderate persistent type: symptoms occur chronically and daily activities or sleep is impaired once or more in a week, with a nocturnal attack occurring once or more in a week; and (iv) severe persistent type: asthma symptoms persist nearly everyday and often aggravate, and daily activities are limited, with nocturnal symptoms being prevalent.

As etiology of bronchial asthma, it is considered that a susceptible person experiences airway inflammation induced by various pathogenic factors (risk factors), for example, allergen exposure, occupational irritating substances, airway viral infection, airway irritating substances, passive smoking and the like, and as a result acquires long persisting airway reactivity accentuation (airway hypersensitivity). Cases of bronchial asthma are classified into the atopic type and the non-atopic type. The most important of the pathogenic factors is the atopic type, the majority of infantile cases of asthma being of the atopic type. The mechanism for atopic type asthma is as described below. The IgE antibody bound to the surface of mast cells, which are a type of Immune cells, reacts with an allergen (allergy-causing substance) to produce chemical mediators such as histamine, leukotriene, and prostaglandin from mast cells. Subsequently, these chemical mediators constrict bronchial smooth muscle to cause convulsions, and also induce mucosal edema to cause bronchial stenosis. This is a reaction called an immediate reaction, and this symptom maximizes 15 minutes to 30 minutes after allergen inhalation and resolves in about 1 hour. Subsequently, about half the patients again experience attacks due to late reactions 4 to 8 hours later.

The late attacks are airway smooth muscle constriction and airway mucosal inflammation caused by eosinophils. Eosinophils are gathered in the bronchia by Eosinophil Chemotactic Factor (ECF) released from mast cells, platelet activating factors, and cytokines produced by helper T cells, such as IL-5. Then, the eosinophils release granular proteins such as major basic protein (MBP), eosinophil peroxidase (EPO), eosinophil cationic protein (ECP), and eosinophil-derived neurotoxin (EDN), and bioactive mediators such as LTC4, LTD4, and PAF; the actions thereof cause smooth muscle constriction and mucosal inflammation.

On the other hand, regarding the mechanism for non-atopic type asthma, no detailed mechanism of onset has been clarified, but possible inducers include airway infection, as well as cold air, air pollutants, stress and the like. There are some hypotheses concerning the route of development of attacks, including a route medicated by the parasympathetic nerves, a route mediated by mast cells, a route based on direct action on lymphocytes, and the like. When exposure to a sensitizing factor is continued, airway inflammation and airway reactivity are persisted and aggravated. The majority of asthma attack inducing factors, like pathogenic factors, are allergens, airway viral infection, and airway stimulants, and trigger attacks and at the same time make airway inflammation to persist.

Regarding the treatment of bronchial asthma, it is reportedly difficult to remove the cause of the disease and achieve healing because this is a predispositional disease. Therapeutic approaches can be roughly divided mainly into environmental controls such as avoidance and removal of airway inflammation inducing factors and attack inducing factors, and drug therapy. In drug therapy, in particular, drugs such as adrenocortical hormone drugs (steroid drugs), anti-allergic drugs, β2 adrenaline stimulants (β2 stimulants), and theophylline are currently used as prophylactic drugs or symptomatic first-aid drugs.

In addition, in relation to symptomatic therapy for autoimmune diseases, a method of ameliorating the mood of patients with autoimmune diseases such as Myasthenia Gravis, Guillain-Barr like syndrome, and Behcet's disease by intravenous administration of a mixture of isoleucine, leucine, lysine, methionine, phenylalanine, tryptophan, valine, alanine, arginine, histidine, proline, serine, glycine, and cysteine has been reported (the specification for US Patent Application Publication No. 2000/6274612). However, in the specification, there is no description that the above-described amino acids ameliorate symptoms of allergic diseases caused by Th2 cell-polarized activation, such as asthma. Hence, the specification mentions an evaluation of the improvement of QOL but does not show that the allergic inflammation per se is ameliorated or symptom aggravation can be prevented.

Furthermore, the present applicant has disclosed a therapeutic/prophylactic agent for inflammatory diseases comprising ornithine and a branched amino acid as active ingredients (pamphlet for WO 02/060431). The inflammation disclosed therein is a type of inflammation caused by immune diseases classified under Th1 diseases such as rheumatoid arthritis. On the other hand, allergic diseases and the like including asthma belong to the type of Th2 diseases, the mechanism of the onset thereof being completely opposite to that of Th1 diseases. Therefore, nothing is known about the efficacy of ornithine and branched amino acids against the inflammation caused by Th2 diseases.

### Disclosure of Invention

In the drug therapy for bronchial asthma and the like, the drugs used are divided into prophylactic drugs and symptomatic first-aid drugs, and their kinds, doses, administration methods etc. vary depending on the levels of patient symptoms and attacks. Multiple onsets of attacks not only produce burdens such as dyspnea, but also lead to aggravation of the condition due to repeats of bronchial mucosal damage and remodeling. It is important to secure the management of chronic asthma to prevent attacks from developing. Therefore, for a patient to continue to take a drug for a long time, the drug must be one that produces a low prevalence of side effects and permits long-term administration.

However, all of currently used steroid drugs, anti-allergic drug, β2 stimulants, theophylline and the like produce side effects. For this reason, when these drugs are administered for a long time or administered at high doses in the event of symptom aggravation or serious attacks, a wide variety of side effects are anticipated. To obtain a dose reduction effect for steroid drugs, coadministration with anti-allergic drugs is performed, but because anti-allergic drugs also produce side effects as described above, mitigation of side effects has not been achieved. As examples of side effects of steroid drugs, irritating feeling in the throat and development of fungi such as candida can be mentioned. As side effects of anti-allergic drugs, gastrointestinal symptoms (abdominal pain, nausea, vomiting, diarrhea), hypersensitivities (eruption, urticaria and the like), cystitis-like symptoms (pollakiuria, urodynia, hematuria and the like), thrombocytopenia and the like can be mentioned. As side effects of β2 stimulants, hypertension, facial pallor, headache, excitation, insomnia and the like can be mentioned; as side effects of theophylline, hypersensitive symptoms (eruption and the like), gastrointestinal symptoms, nausea, excitation, headache and the like can be mentioned. There are some cases in which laboratory examination is performed periodically during the drug administration period in order to avoid the risk due to these side effects, and this examination also exerts physical, mental, and economic burdens on the patient. Because safety in fetuses has not been established, anti-allergic drugs for oral ingestion are, as a rule, not used during pregnancy.

Under these circumstances, it is an object of the present invention to provide a drug and food that are capable of preventing or treating an allergic disease caused by Th2 cell-polarized activation, and that can be safely ingested routinely.

The present inventors diligently investigated to accomplish the above-described object, and found that by orally administering a branched amino acid, glycine, serine, arginine (these are hereinafter also referred to as "a particular amino acid"), a keto acid thereof or a salt thereof to an asthma model mouse, the Th1/Th2 balance with Th2 being predominant at the inflammation site in the asthma model mouse is corrected, accumulation of immune cells at the inflammation site, hypertrophy of tracheal epithelial cells, mucus secretion accentuation and the like observed in bronchial asthma are suppressed and the respiratory function is ameliorated, that these ameliorating effects on allergic symptoms are enhanced by coadministration of ornithine or a salt thereof, and that by combining the above-described particular amino acid and the like and an existing anti-inflammatory drug (for example, a steroid drug), the dose of the anti-inflammatory drug can be reduced. Furthermore, the present inventors found that the above-described particular amino acid and the like were very highly effective in the prevention or treatment of an allergic disease, particularly bronchial asthma or pulmonary fibrosis that accompanies bronchial asthma, produced almost no side effects, and are suitable for pharmaceuticals (including infusions, nutritive tonics and the like) and foods (including medical foods, health foods, foods for specified health uses and the like), and developed the present invention on the basis of these findings. Accordingly, the present invention encompasses the following contents:
(1) A prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient.
(2) The prophylactic/therapeutic agent described in (1), wherein the aforementioned allergic disease is bronchial asthma.
(3) The prophylactic/therapeutic agent described in (1), wherein the aforementioned allergic disease is pulmonary fibrosis that accompanies bronchial asthma.
(4) The prophylactic/therapeutic agent described in any one of (1) to (3), wherein the aforementioned branched amino acid is selected from the group consisting of valine, leucine and isoleucine.
(5) The prophylactic/therapeutic agent described in any one of (1) to (4), wherein the aforementioned keto acid is selected from the group consisting of 2-oxoisovaleric acid, 3-methyl-2-oxovaleric acid, 4-methyl-2-oxovaleric acid and glyoxylic acid.
(6) The prophylactic/therapeutic agent described in any one of (1) to (5), which further comprises ornithine or a salt thereof.
(7) A combination drug comprising a combination of the prophylactic/therapeutic agent described in any one of (1) to (6) and an anti-inflammatory drug.
(8) The combination drug described in (7), wherein the aforementioned anti-inflammatory drug is a steroid drug.
(9) A food for the prevention/treatment of an allergic disease caused by Th2 cell-polarized activation, which comprises at least one kind of compound selected from the group consisting of a branched amino acid, glycine, serine, arginine, a keto acid thereof and a salt thereof.
(10) The food described in (9), which is a food with health claims or a dietary supplement.
(11) The food described in (10), wherein the aforementioned food with health claims is a food for specified health uses or a food with nutrient function claims.
(12) The food described in any one of (9) to (11), wherein the aforementioned branched amino acid is selected from the group consisting of valine, leucine and isoleucine.
(13) The food described in any one of (9) to (12), wherein the aforementioned keto acid is selected from the group consisting of 2-oxoisovaleric acid, 3-methyl-2-oxovaleric acid, 4-methyl-2-oxovaleric acid and glyoxylic acid.
(14) The food described in any one of (9) to (13), which further comprises ornithine or a salt thereof.
(15) A commercial package comprising a composition comprising at least one kind selected from the group consisting of a branched amino acid, glycine, serine, arginine, a keto acid thereof and a salt thereof as an active ingredient, and a printed matter bearing the statement that the composition can be used, or should be used, for the prevention/treatment of an allergic disease caused by Th2 cell-polarized activation.
(16) A commercial package comprising a combination drug comprising a composition comprising at least one kind selected from the group consisting of a branched amino acid, glycine, serine, arginine, a keto acid thereof and a salt thereof as an active ingredient and an anti-inflammatory drug, and a printed matter bearing the statement that the combination drug can be used, or should be used, for the prevention/treatment of an allergic disease caused by Th2 cell-polarized activation.
(17) A commercial package comprising a food comprising at least one kind of compound selected from the group consisting of a branched amino acid, glycine, serine, arginine, a keto acid thereof and a salt thereof, and a printed matter bearing the statement that the food can be used, or should be used, for the prevention/treatment of an allergic disease caused by Th2 cell-polarized activation.
(18) A use of a branched amino acid, glycine, serine, arginine, a keto acid thereof or a salt thereof for producing a prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient.
(19) A use of at least one kind of compound selected from the group consisting of a branched amino acid, glycine, serine, arginine, a keto acid thereof and a salt thereof, and an anti-inflammatory drug, for producing a combination drug comprising a combination of a prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient, and an anti-inflammatory drug.
(20) A use of a branched amino acid, glycine, serine, arginine, a keto acid thereof and a salt thereof for producing a food for the prevention/treatment of an allergic disease caused by Th2 cell-polarized activation, which comprises at least one kind of compound selected from the group consisting of a branched amino acid, glycine, serine, arginine, a keto acid thereof and a salt thereof.
(21) The use described in any one of (18) to (20), wherein the aforementioned allergic disease is bronchial asthma.
(22) The use described in any one of (18) to (20), wherein the aforementioned allergic disease is pulmonary fibrosis that accompanies bronchial asthma.
(23) The use described in any one of (18) to (22), wherein the aforementioned branched amino acid is selected from the group consisting of valine, leucine and isoleucine.
(24) The use described in any one of (18) to (23), wherein the aforementioned keto acid is selected from the group consisting of 2-oxoisovaleric acid, 3-methyl-2-oxovaleric acid, 4-methyl-2-oxovaleric acid and glyoxylic acid.
(25) The use described in any one of (18) to (24), which further comprises ornithine or a salt thereof.
(26) A prophylactic/therapeutic method for an allergic disease caused by Th2 cell-polarized activation, which comprises administering an effective amount of at least one kind selected from the group consisting of a branched amino acid, glycine, serine, arginine, a keto acid thereof and a salt thereof to a subject of administration.
(27) A prophylactic/therapeutic method for an allergic disease caused by Th2 cell-polarized activation, which comprises administering an effective amount of at least one kind selected from the group consisting of a branched amino acid, glycine, serine, arginine, a keto acid thereof and a salt thereof, and an anti-inflammatory drug, to an subject of administration.
(28) A prophylactic/therapeutic method for an allergic disease caused by Th2 cell-polarized activation, which comprises ingesting a food comprising at least one kind of compound selected from the group consisting of a branched amino acid, glycine, serine, arginine, a keto acid thereof and a salt thereof.
(29) The method described in any one of (26) to (28), wherein the aforementioned allergic disease is bronchial asthma.
(30) The method described in any one of (26) to (28), wherein the aforementioned allergic disease is pulmonary fibrosis that accompanies bronchial asthma.
(31) The method described in any one of (26) to (30), wherein the aforementioned branched amino acid is selected from the group consisting of valine, leucine and isoleucine.
(32) The method described in any one of (26) to (31), wherein the aforementioned keto acid is selected from the group consisting of 2-oxoisovaleric acid, 3-methyl-2-oxovaleric acid, 4-methyl-2-oxovaleric acid and glyoxylic acid.
(33) The method described in any one of (26) to (32), which further comprises ornithine or a salt thereof.

### Brief Description of the Drawings

Figure 1 is a drawing showing the asthma suppressive effects observed when 0.12 to 15 mM aqueous solutions of valine were administered to ovalbumin-induced asthma model mice by the free water drinking method.
Figure 2 is a drawing showing the asthma suppressive effects observed when a 0.9 mM aqueous solution of leucine was administered to ovalbumin-induced asthma model mice by the free water drinking method.
Figure 3 is a drawing showing the asthma suppressive effects observed when a 0.45 mM aqueous solution of isoleucine was administered to ovalbumin-induced asthma model mice by the free water drinking method.
Figure 4 is a drawing showing the asthma suppressive effects observed when 3 to 45 mM aqueous solutions of arginine were administered to ovalbumin-induced asthma model mice by the free water drinking method.
Figure 5 is a drawing showing the asthma suppressive effects observed when 0.6 to 15 mM solutions of serine or glycine were administered to ovalbumin-induced asthma model mice by the free water drinking method.
Figure 6 is a drawing showing the results of an airway hypersensitivity test observed when a 3 mM aqueous solution of valine was administered to ovalbumin-induced asthma model mice by the free water drinking method.
Figure 7 is a drawing showing the immune cell infiltration suppressive effects in lung tissue observed when a 3 mM aqueous solution of valine and a 15 mM aqueous solution of ornithine were administered to ovalbumin-induced asthma model mice by the free water drinking method.
Figure 8 is a drawing showing the asthma suppressive effects observed when a 15 mM aqueous solution of valine and dexamethasone were coadministered to ovalbumin-induced asthma model mice.
Figure 9 is a drawing showing the allergic symptom ameliorating effects observed when a mixture of valine and ornithine hydrochloride (both at 15 mM) was administered to ovalbumin-induced asthma model mice by the free water drinking method.
Figure 10 is a drawing showing the allergic symptom ameliorating effects observed when a mixture of valine and arginine (both at 15 mM) was administered to ovalbumin-induced asthma model mice by the free water drinking method.
Figure 11 is a drawing showing the allergic symptom ameliorating effects observed when valine (0.6, 3, 15 g/L) was administered to ovalbumin-induced asthma model mice by Oral administration method.
Figure 12 is a drawing showing the allergic symptom ameliorating effects observed when valine (0.6, 15 g/L) was administered to ovalbumin-induced asthma model mice by the inhalation administration method.
Figure 13 is a drawing showing the anti-inflammatory effects observed when valine (15 g/L) and dexamethasone (10 mg%) were coadministered to ovalbumin-induced pulmonary fibrosis model mice by inhalation.
Figure 14 is a drawing showing the airway resistance ameliorating effects observed when valine (15 g/L) and dexamethasone (10 mg%) were coadministered to ovalbumin-induced pulmonary fibrosis model mice by inhalation.
Figure 15 is a drawing showing the pulmonary fibrosis suppressive effects observed when valine (15 g/L) and dexamethasone (10 mg%) were coadministered to ovalbumin-induced pulmonary fibrosis model mice by inhalation.

### Best Mode for Carrying out the Invention

Modes of embodiment of the present invention are hereinafter described.

The prophylactic/therapeutic agent of the present invention refers to a prophylactic/therapeutic agent for an allergic disease caused by Th2 cell-polarized activation, which comprises at least one kind selected from a branched amino acid, glycine, serine, arginine, a keto acid thereof and a salt thereof (these are hereinafter also referred to as "a particular amino acid and the like") as an active ingredient. The combination drug of the present invention refers to a combination drug for the prevention/treatment of an allergic disease caused by Th2 cell-polarized activation, which comprises a combination of the above-described prophylactic/therapeutic agent and an anti-inflammatory drug. Furthermore, the food of the present invention refers to a food for the prevention/treatment of an allergic disease caused by Th2 cell-polarized activation, which comprises at least one kind of compound selected from the group consisting of a branched amino acid, glycine, serine, arginine, a keto acid thereof and a salt thereof.

In the present invention, "a branched amino acid" refers to a hydrophobic essential amino acid having a branched alkyl chain in a side chain thereof; specifically, valine, leucine, isoleucine and the like can be mentioned.

In the present invention, "a keto acid" refers to a keto acid obtained by subjecting the above-described amino acid to a deamination reaction, which can be converted to the above-described amino acid in a living organism by an amino substitution reaction (J. Nutrition 101: 1165-1168, 1971). As such keto acids, 2-oxoisovaleric acid, 3-methyl-2-oxovaleric acid, 4-methyl-2-oxovaleric acid, glyoxylic acid and the like can be mentioned.

The "salt" as used in the present invention is preferably a pharmacologically acceptable salt of an amino acid or keto acid; as examples of such salts, a salt with an inorganic acid, a salt with an organic acid, and a salt with an inorganic base can be mentioned. As example of the salt of an inorganic acid, salts of halogenated hydroacids (hydrochloric acid, hydrobromic acid, hydroiodic acid and the like), sulfuric acid, nitric acid, phosphoric acid and the like can be mentioned. As the salt of an organic acid, salts of formic acid, acetic acid, propionic acid, oxalic acid, succinic acid, maleic acid, fumaric acid, citric acid, glutamic acid, aspartic acid and the like can be mentioned. As the salt of an inorganic base, salts of alkali metals such as lithium, sodium, and potassium, salts of alkaline earth metals such as calcium and magnesium, ammonium salts and the like can be mentioned.

In the present invention, a branched amino acid, glycine, serine, arginine, a keto acid thereof or a salt thereof can be used in combination of two kinds or more; as examples of a suitable combination thereof, valine and arginine, valine and serine, valine and glycine, leucine and arginine, and isoleucine and arginine can be mentioned. Of these, valine and arginine, leucine and arginine, and isoleucine and arginine are more preferable because the prophylactic or therapeutic effect on an allergic disease is further enhanced.

Furthermore, the present invention may further comprise ornithine or a salt thereof as an active ingredient. The "salt" is preferably a pharmacologically acceptable salt; as examples of such salts, a salt with an inorganic acid, a salt with an organic acid, and a salt with an inorganic base can be mentioned. As specific examples thereof, the above-described ones can be mentioned.

The branched amino acid, glycine, serine, arginine and ornithine used may be any animal- or plant-derived natural substance, or a product obtained by a chemical synthesis method or a fermentation method. These may be optical isomers or racemates. Of these, L-forms are suitably used because they occur naturally, or because they are bioproteins. The same applies to the keto acids and salts thereof.

An allergic disease in the present invention refers to a disease involved by an allergic reaction, and an allergic disease caused by Th2 cell-polarized activation refers to a disease wherein Th2 cell reactions are extremely predominant over Th1 cell responses. As specific examples of the allergic disease, bronchial asthma, pulmonary fibrosis that accompanies bronchial asthma, allergic rhinitis, atopic dermatitis and the like can be mentioned. Preferably, bronchial asthma and pulmonary fibrosis that accompanies bronchial asthma can be mentioned.

The prophylactic/therapeutic agent of the present invention acts to correct the Th1/Th2 balance with Th2 being predominant at the inflammation site. Therefore, the prophylactic/therapeutic agent of the present invention is useful as a prophylactic/therapeutic agent for an allergic disease caused by Th2 cell-polarized activation, and is capable of relieving bronchial asthma symptoms, suppressing aggravation, ameliorating the symptoms, and preventing the recurrence of bronchial asthma or pulmonary fibrosis that accompanies bronchial asthma. Hence, by orally administering a very low dose of a particular amino acid and the like to an asthma model mouse, a reduction in the accumulation of eosinophils at the inflammation site, and an increase in the IFN-γ/IL-4 ratio in the BAL Fluid were observed. With a particular amino acid and the like, an inflammatory state in which the Th1/Th2 balance is affected with Th2 being predominant at the airway inflammation site can be corrected, and accumulation of immune cells at the inflammation site, hypertrophy of tracheal epithelial cells, mucus secretion accentuation, pulmonary fibrosis and the like observed in bronchial asthma are suppressed. Therefore, the prophylactic/therapeutic agent of the present invention has a mechanism of pharmacological effect different from that of conventional anti-inflammatory drugs such as steroid drugs, and drugs such as histamine H1 antagonists, leukotriene antagonists, and long-acting bronchodilators (sustained-release theophylline drugs, long-acting β2 stimulants and the like).

In the prevention and/or treatment of an allergic disease caused by Th2 cell-polarized activation, such as bronchial asthma, an effective amount of a particular amino acid and the like can be administered to a subject of administration. In this case, the particular amino acid and the like can be prepared and administered in the form of various commonly known pharmaceutical preparations, for example, various dosage forms such as oral administration (oral medicines), intraperitoneal administration, percutaneous administration, inhalation administration (inhalation administration as mentioned herein refers to a method of delivering a high dose of a powdered or aerosolized drug to lung tissue through the trachea by means of the force of a compressed gas or its own inhaling force), nasal drops, eye drops, and the like. However, the method of administration is not limited to these methods of administration. As used herein, an effective amount refers to an amount that is sufficient to produce a desired prophylactic or therapeutic effect; as the subject of administration, an individual suffering an allergy caused by Th2 cell-polarized activation (for example, mammals such as humans, cattle, horses, dogs, mice, and rats; the same applies below), an individual possibly suffering the allergy, and the like can be mentioned.

As examples of forms suitable for administration of pharmaceutical preparations, appropriate solid or liquid forms can be mentioned; specifically, granules, powders, triturates, coated tablets, tablets, (micro)capsules, suppositories, syrups, juices, suspending agents, emulsifying agents, dripping agents, and liquids for injection (including infusions, nutritive drugs and the like) can be mentioned, and these can be prepared as sustainable-release preparations. To prepare the prophylactic/therapeutic agent of the present invention in these various forms of pharmaceutical preparations, commonly known methods can be adopted as appropriate.

Furthermore, the prophylactic/therapeutic agent of the present invention may further comprise various pharmaceutically acceptable substances for pharmaceutical making (auxiliaries). A substance for pharmaceutical making can be chosen as appropriate according to the form of the preparation; for example, excipients, diluents, additives, disintegrants, binders, coating agents, lubricants, sliding agents, glazing agents, flavoring agents, sweetening agents, solubilizers and the like can be used. Specifically, magnesium carbonate, titanium dioxide, lactose, mannitol and other saccharides, talc, cow's milk protein, gelatin, starch, cellulose and derivatives thereof, animal or vegetable oils, polyethylene glycol, solvents (for example, sterile water and monohydric or polyhydric alcohols (for example, glycerol)) and the like can be mentioned.

Because the prophylactic/therapeutic agent of the present invention has a particular amino acid and the like as an active ingredient, the prevalence of side effects is extremely low, or nearly zero, compared to currently used anti-inflammatory drugs and the like. For example, the prophylactic/therapeutic agent of the present invention can be used in place of inhaled steroid drugs if the patient has mild symptoms, and in the case of relatively mild symptoms of the mild intermittent type or mild persistent type, not only adults but also children and pregnant women are able to take the prophylactic/therapeutic agent of the present invention for a long time without being annoyed by side effects.

In the prophylactic/therapeutic agent of the present invention, a particular amino acid and the like may be administered as is alone as a drug, but when another drug is coadministered, the dose of the other drug can be reduced, or the effect thereof can be increased. Specifically, when an inhaled steroid drug is coadministered with the prophylactic/therapeutic agent of the present invention, the dose thereof can be reduced. Provided that the condition of the patient is moderate, when an oral steroid drug is coadministered with the prophylactic/therapeutic agent of the present invention, a reduction in the dose thereof is expectable.

Furthermore, when an oral steroid drug and an anti-allergic drug are coadministered with the prophylactic/therapeutic agent of the present invention, the side effects of each can be mitigated, or resolved.

On the other hand, when a bronchodilator is coadministered with the prophylactic/therapeutic agent of the present invention, the effect of the bronchodilator is enhanced, and the dose thereof can be reduced.

As drugs that exhibit an additive or synergistic effect when coadministered with the prophylactic/therapeutic agent of the present invention, ephedrine hydrochloride, adrenaline, isoproterenol, orciprenaline sulfate, trimetoquinol hydrochloride, clorprenaline hydrochloride, salbutamol sulfate, terbutaline sulfate, hexoprenaline sulfate, fenoterol hydrobromide, tulobuterol hydrochloride, procaterol hydrochloride, clenbuterol hydrochloride, mabuterol hydrochloride, and formoterol fumarate, which are therapeutic drugs for bronchial asthma and β2 stimulants; aminophylline, choline theophylline, theophylline sustained-release preparations, diprophylline, diprophylline mixture drugs, proxyphylline, and proxyphylline mixture drugs, which are theophylline drugs; Aldecin, Becotide, Flutide, Pamilquat, beclometasone, fluticasone, and butesonide, which are steroid drugs; sodium cromoglicate, tranilast, amlexanox, repirinast, ibudilast, pemirolast potassium, tazanolast, ketotifen fumarate, azelastine hydrochloride, mequitazine, epinastine hydrochloride, ozagrel hydrochloride, seratrodast, pranlukast hydrate, zafirulukast, montelukast, and suplatast tosilate, which are anti-allergic drugs; and the like can be mentioned.

In the prophylactic/therapeutic agent of the present invention, the oral dose of a particular amino acid and the like as an active ingredient is adjusted according to the illness targeted in the present invention (for example, bronchial asthma), condition of the disease, the patient's body weight, sex, age, predisposition, physical condition and the like. The dose of a particular amino acid and the like per day (assuming an adult weighing 60 kg) is, for example, preferably about 1 mg to 60 g, more preferably 500 mg to 15 g. In the case of other mammals and children, an amount determined by converting the above-described dose to a body weight ratio for an adult and the like can be administered or ingested.

In the case of non-oral administration, for example, administration of a solution containing a particular amino acid and the like by inhalation or nasal drop spraying, and as an injection, and the like are possible. In the case of non-oral administration, about 1/2 to 1/20 of the above-described oral dose can be used, depending on the patient condition, form of the preparation and the like. Regarding the frequency of administration per day, the prophylactic/therapeutic agent of the present invention can be taken in several divided portions according to the condition of the illness and the form of the drug and the like.

The combination drug of the present invention comprises a combination of the above-described prophylactic/therapeutic agent and an anti-inflammatory drug. The combination drug of the present invention acts to correct the Th1/Th2 balance with Th2 being predominant at the inflammation site. Therefore, the combination drug of the present invention is useful as a prophylactic/therapeutic agent for an allergic disease caused by Th2 cell-polarized activation, and allows a reduction in the dose of the anti-inflammatory drug. As the anti-inflammatory drug, steroid drugs (inhalation, oral), anti-allergic drugs (inhalation, oral), leukotriene/thromboxane receptor antagonists and the like can be mentioned. Preferably, steroid drugs can be mentioned.

The combination drug of the present invention comprises a combination of the prophylactic/therapeutic agent of the present invention and an anti-inflammatory drug, and may be any one, as long as it allows combining the prophylactic/therapeutic agent and the anti-inflammatory drug at the time of administration. Therefore, the combination drug of the present invention may be a single preparation obtained by simultaneously preparing the prophylactic/therapeutic agent and the anti-inflammatory drug, or a combination of 2 kinds of preparations obtained by separately preparing the prophylactic/therapeutic agent and the anti-inflammatory drug, as long as it allows combining the prophylactic/therapeutic agent and the anti-inflammatory drug at the time of administration. The dosage form is not subject to limitation; for example, (1) administration as a composition comprising the prophylactic/therapeutic agent and the anti-inflammatory drug, that is, a single preparation, (2) simultaneous administration of two kinds of preparations obtained by separately preparing the prophylactic/therapeutic agent and the anti-inflammatory drug via the same route of administration, (3) administration of two kinds of preparations obtained by separately preparing the prophylactic/therapeutic agent and the anti-inflammatory drug via the same route of administration at a time lag (for example, administration in the order of the prophylactic/therapeutic agent and the anti-inflammatory drug, or administration in the reverse order), (4) simultaneous administration of two kinds of preparations obtained by separately preparing the prophylactic/therapeutic agent and the anti-inflammatory drug via different routes of administration, (5) administration of two kinds of preparations obtained by separately preparing the prophylactic/therapeutic agent and the anti-inflammatory drug via different routes of administration at a time lag (for example, administration in the order of the prophylactic/therapeutic agent and the anti-inflammatory drug, or administration in the reverse order) and the like can be mentioned.

In the combination drug of the present invention, the dose of the anti-inflammatory drug (in the case of a steroid drug) per day (assuming an adult weighing 60 kg) is, for example, preferably about 10 µg to 10 mg, more preferably 100 µg to 1 mg.

The content of the anti-inflammatory drug (in the case of a steroid drug) in the combination drug of the present invention is normally 0.00005 to 0.005% by weight, preferably 0.0001 to 0.01% by weight, more preferably 0.0005 to 0.1% by weight.

Although the addition ratio of the prophylactic/therapeutic agent and the anti-inflammatory drug (in the case of a steroid drug) in the combination drug of the present invention is not subject to limitation, the anti-inflammatory drug can be contained in a ratio by weight of preferably about 1/50000 to 1/50, more preferably about 1/10000 to 1/100, still more preferably about 1/5000 to 1/500, to 1 of a particular amino acid and the like contained in the prophylactic/therapeutic agent of the present invention.

In the combination drug of the present invention, the dose of the anti-inflammatory drug (in the case other than steroid drugs) per day (assuming an adult weighing 60 kg) is, for example, preferably about 1 mg to 10 g, more preferably 10 mg to 1 g.

The content of the anti-inflammatory drug (in the case other than steroid drugs) in the combination drug of the present invention is normally 0.005 to 95% by weight, preferably 0.01 to 80% by weight, more preferably 0.05 to 70% by weight.

Although the addition ratio of the prophylactic/therapeutic agent and the anti-inflammatory drug (in the case other than steroid drugs) in the combination drug of the present invention is not subject to limitation, the anti-inflammatory drug can be contained in a ratio by weight of preferably about 1/15000 to 20, more preferably about 1/5000 to 10, still more preferably about 1/1500 to 2, to 1 of the particular amino acid and the like contained in the prophylactic/therapeutic agent of the present invention.

The drug of the present invention in the forms of preparations exemplified above should of course comprise the above-described active ingredient in an amount effective for exhibiting the pharmacological effect thereof. The dose of the drug of the present invention is the same as described above. Various ingredients other than the essential active ingredient used in the present invention can also be used; in this case as well, a desired preparation can be prepared on the basis of the ingredients used, by utilizing known pharmaceutical making techniques, and according to various dosage forms.

As the food of the present invention, a particular amino acid and the like may be ingested as a food as is in the same manner as the above-described drug, but to render the particular amino acid and the like more readily ingestible, it may be prepared as a processed food supplemented with ordinary food materials and additives such as seasonings and flavoring agents. Here, as the food in the present invention, in addition to common foods, including what are called health foods, medical foods, foods with health claims, foods for specified health uses, and foods with nutrient function claims stipulated in the food with health claims system of the Ministry of Health, Labor and Welfare, can be mentioned, and dietary supplements can also be mentioned.

As the form of the food, any forms used for ordinary foods and the like, such as powders, granules, fine granules, tablets, capsules, liquids, and jellies, are possible. A particular amino acid and the like can be ingested as an ingredient added to existing foods, for example, drinks, refreshing beverages, yogurt, malt syrups, jellies, milk beverages and the like.

When a particular amino acid and the like is ingested in the form of a food, the amount ingested is adjusted according to the illness targeted in the present invention (for example, bronchial asthma), the condition of the disease, the patient's body weight, sex, age, predisposition, physical condition and the like.

The amount of a particular amino acid and the like ingested per day (assuming an adult weighing 60 kg) is, for example, preferably about 1 mg to 60 g, more preferably 500 mg to 15 g. Such an amount ingested can be administered at one time or in several divided portions. In the case of other mammals and children, an amount determined by converting the above-described amount ingested to a body weight ratio for an adult and the like can be ingested.

In the prophylactic/therapeutic agent, combination drug or food of the present invention, when valine is used in combination with leucine, isoleucine, glycine, serine, arginine, a keto acid thereof or a salt thereof (hereinafter referred to as "an amino acid other than valine etc."), the amount thereof used can be chosen as appropriate with reference to the above-described dose and the like. In this case, the blending ratio of valine and an amino acid other than valine etc. is not subject to limitation, but the amino acid other than valine etc. can be used in a ratio by weight of preferably about 0.1 to 10, more preferably about 1 to 5, still more preferably about 1 to 2, to 1 of valine. When ornithine is further contained, the blending ratio of ornithine and a particular amino acid and the like is not subject to limitation, but, for example, ornithine can be contained in a ratio by weight of preferably about 0.01 to 100, more preferably about 0.05 to 50, still more preferably about 0.1 to 20, to 1 of valine.

The particular amino acid and the like of the present invention can also be used as a quasi-drug and the like comprising the same. A quasi-drug refers to an article positioned between a pharmaceutical and a cosmetic, less potent than pharmaceuticals and not intended for the diagnosis or treatment of disease, having clearer indications than those of cosmetics, and intended for prevention without causing side effects. As the form of the quasi-drug, bathing agents, creams, emulsions, hand creams, lotions, medicinal soaps (including face cleansing agent), shampoos, rinses, eyedrops, nasal drops and the like can be mentioned, but these are not to be construed as limiting.

The present invention encompasses a commercial package comprising a composition comprising a particular amino acid and the like as an active ingredient, and a printed matter bearing the statement that the composition can be used, or should be used, for the prevention/treatment of an allergic disease caused by Th2 cell-polarized activation, and the like.

The present invention also provides a prophylactic/therapeutic method for an allergic disease caused by Th2 cell-polarized activation, and the method comprises a step for administering an effective amount of a particular amino acid and the like, or an effective amount of a particular amino acid and the like and an anti-inflammatory drug, to a subject of administration in need of the prevention/treatment of the disease (for example, human or non-human animal).

### Examples

The present invention is hereinafter described in more detail by means of the following examples, which, however, are not to be construed as limiting the present invention.

### (Example 1)

### <Dose-related changes in the effect of valine administration to ovalbumin-induced asthma model mice>

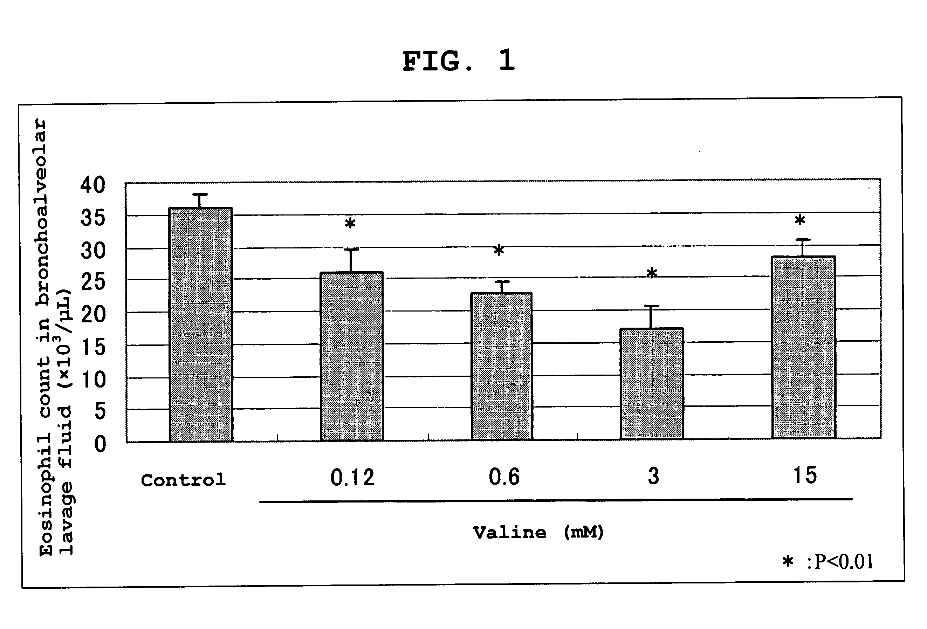

An ovalbumin-induced asthma model was prepared by a conventional method using Balb/c mice, and the drug effect of L-valine (free form) was investigated. The asthma model mice were prepared by utilizing a method well known as a method of preparing an experimental asthma model by sensitizing mice with ovalbumin, and then challenging the mice by suction exposure to an antigen. After 8 µg of ovalbumin and 2 mg of Alum, which is an adjuvant, per mouse were twice (day 0 and day 5) injected intraperitoneally, 1.5% ovalbumin solution was aerosolized with a nebulizer and each mouse was subjected to suction exposure for 30 minutes 3 times (day 12, day 15 and day 20). After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. After 0.12 to 15 mM aqueous solutions of valine were prepared, each was administered by the free water drinking method from day 0 to day 21. As a result, in the control group, the eosinophil count in the bronchoalveolar lavage fluid (BAL Fluid) was 36 x 10³/µL on average. On the other hand, in the valine administration group, the eosinophil count was 26 x 10³/µL, 23 x 10³/µL, 17 x 10³/µL, and 28 x 10³/µL, respectively (see Figure 1). From these results, an asthma suppressive effect of valine administration was confirmed.

### (Example 2)

### <Effect of leucine administration to ovalbumin-induced asthma model mice>

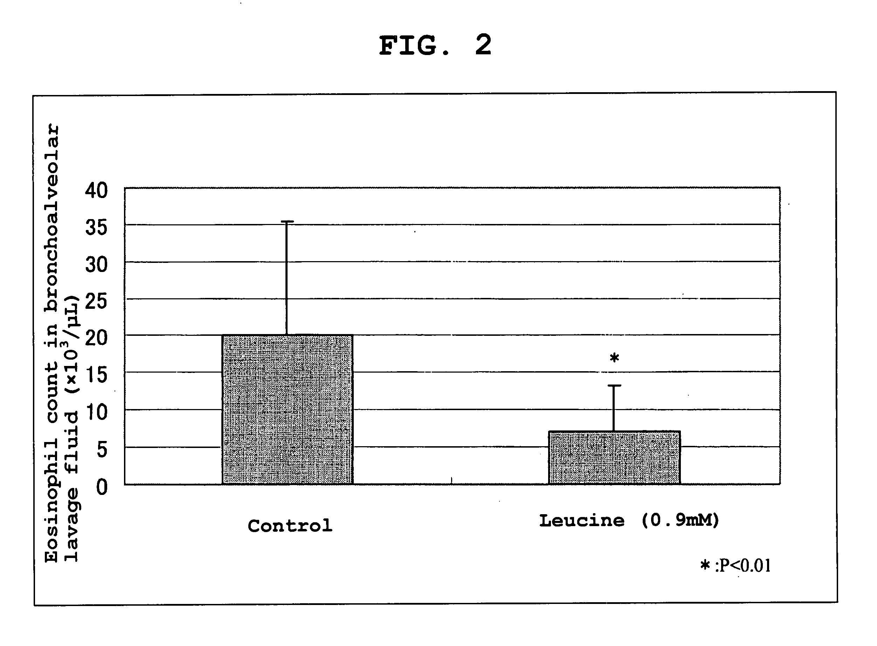

An ovalbumin-induced asthma model was prepared by a conventional method using Balb/c mice, and the drug effect of L-leucine (free form) was investigated. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. After a 0.9 mM aqueous solution of leucine was prepared, it was administered by the free water drinking method from day 0 to day 21. As a result, in the control group, the eosinophil count in the BAL Fluid was 20 x 10³/µL on average. On the other hand, in the leucine administration group, the eosinophil count was 7 x 10³/µL. From these results, an asthma suppressive effect of leucine administration was confirmed (see Figure 2).

### (Example 3)

### <Effect of isoleucine administration to ovalbumin-induced asthma model mice>

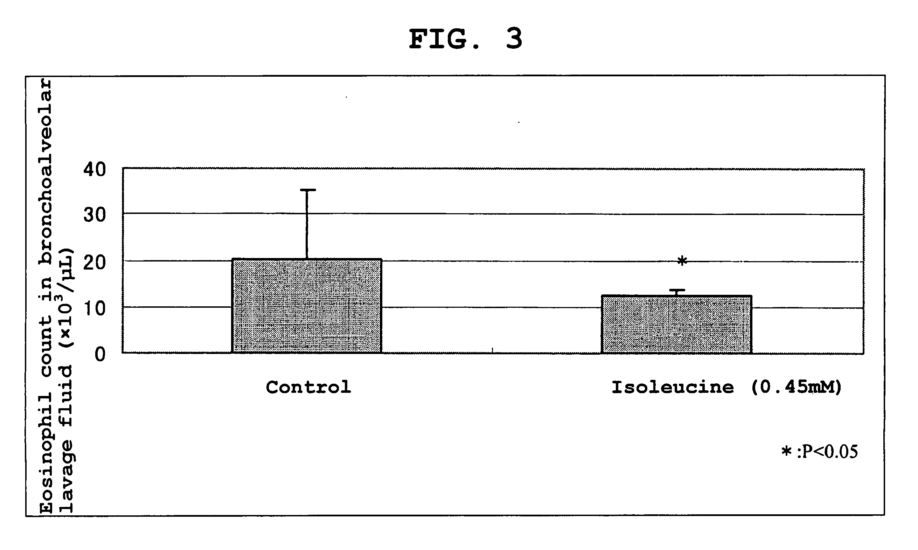

An ovalbumin-induced asthma model was prepared by a conventional method using Balb/c mice, and the drug effect of L-isoleucine (free form) was investigated. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. After a 0.45 mM aqueous solution of isoleucine was prepared, it was administered by the free water drinking method from day 0 to day 21. As a result, in the control group, the eosinophil count in the BAL Fluid was 20 x 10³/µL on average. On the other hand, in the isoleucine administration group, the eosinophil count was 12 x 10³/µL. From these results, an asthma suppressive effect of isoleucine administration was confirmed (see Figure 3).

### (Example 4)

### <Dose-related changes in the effect of arginine administration to ovalbumin-induced asthma model mice>

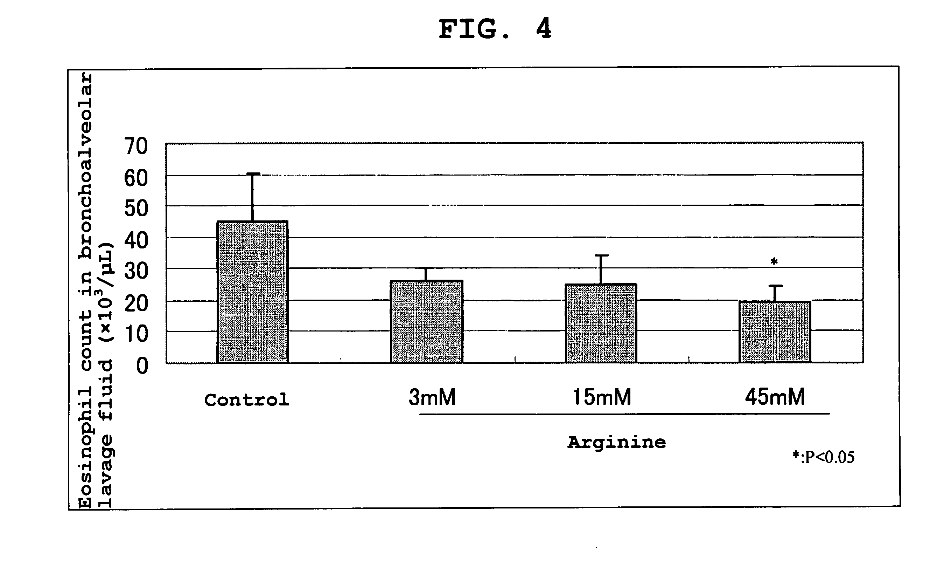

An ovalbumin-induced asthma model was prepared by a conventional method using Balb/c mice, and the drug effect of L-arginine (free form) was investigated. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. After 3 to 45 mM aqueous solutions of arginine were prepared, each was administered by the free water drinking method from day 0 to day 21. As a result, in the control group, the eosinophil count in the BAL Fluid was 45 x 10³/µL on average. On the other hand, in the arginine administration group, the eosinophil count was 26 x 10³/µL, 25 x 10³/µL, and 19 x 10³/µL, respectively. From these results, an asthma suppressive effect of arginine administration was confirmed (see Figure 4).

### (Example 5)

### <Dose-related changes in the effects of serine and glycine administration to ovalbumin-induced asthma model mice>

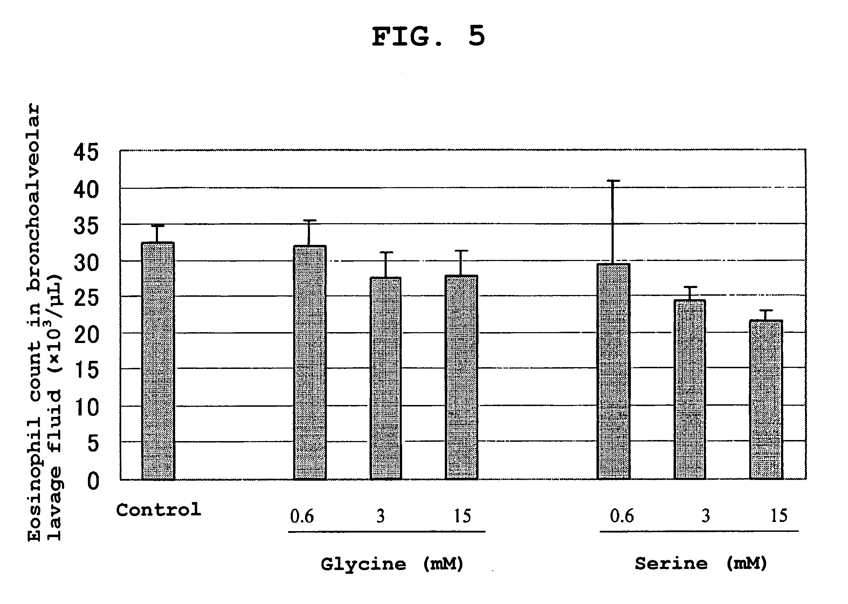

An ovalbumin-induced asthma model was prepared by a conventional method using Balb/c mice, and the drug effects of serine and glycine (free forms) were investigated. After elapse of 24 hours following a third antigen exposure, 7 mL of physiological saline was injected from the bronchia to achieve lavage, and the eosinophil count in the BAL Fluid was measured using an automated blood cell counter and evaluated as an index of localized lung inflammation. After 0.6 to 15 mM aqueous solutions of serine and aqueous solutions of glycine were prepared, each was administered by the free water drinking method from day 0 to day 21. As a result, in the control group, the eosinophil count in the BAL Fluid was 33 x 10³/µL on average. On the other hand, in the serine administration group, the eosinophil count was 29 x 10³/µL, 24 x 10³/µL, and 22 x 10³/µL, respectively. In the glycine administration group, the eosinophil count was 32 x 10³/µL, 27 x 10³/µL, and 28 x 10³/µL, respectively. From these results, asthma suppressive effects of glycine and serine administration were confirmed (see Figure 5).

### (Example 6)

### <Effect of valine administration on the lung function of ovalbumin-induced asthma model mice>

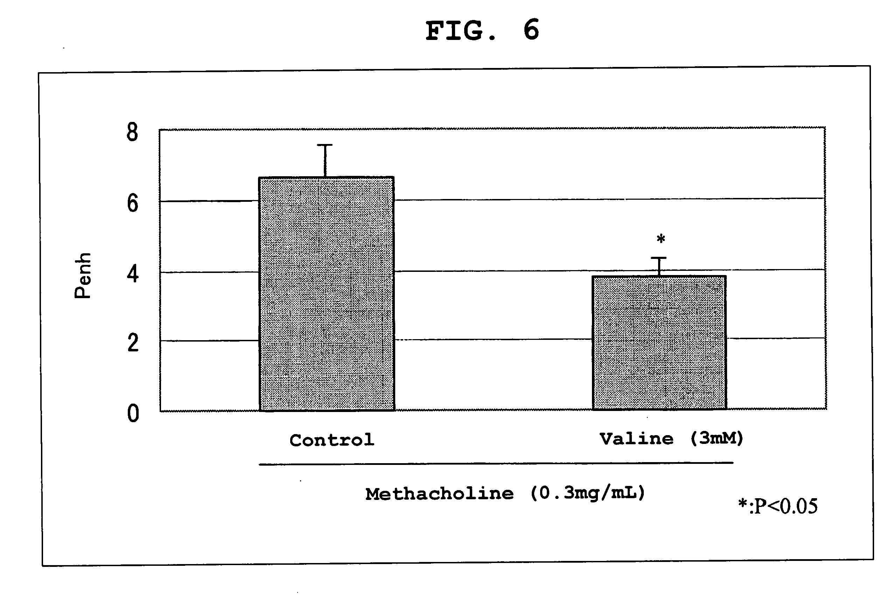

An ovalbumin-induced asthma model was prepared by a conventional method using Balb/c mice, and the drug effect of L-valine (free form) was investigated. After elapse of 24 hours following a third antigen exposure, the animals were allowed to inhale methacholine, which is an airway constricting substance, and an airway hypersensitivity test of the ovalbumin-induced asthma mice was performed using a whole body plethysmography. As an index of airway resistance, the Penh value was used. In the ovalbumin-induced asthma model mice, the Penh value was 6.61; in the group receiving a 3 mM aqueous solution of valine administered by the free water drinking method from day 0 to day 21, the Penh value decreased to 3.77. Hence, a reduction in airway hypersensitivity was observed with valine administration; amelioration of the lung function by valine administration was confirmed (see Figure 6).

### (Example 7)

### <Changes in cytokines in BAL Fluid by valine administration to ovalbumin-induced asthma model mice>

An ovalbumin-induced asthma model was prepared by a conventional method using Balb/c mice, and the drug effect of valine (free form) was investigated. After elapse of 24 hours following a third antigen exposure, 7 mL of physiological saline was injected from the bronchia to achieve lung lavage, and the cytokine concentrations in the BAL Fluid were measured by an ELISA method. After a 0.6 mM aqueous solution of valine was prepared, it was administered by the free water drinking method from day 0 to day 21. The results are shown in Table 1. The IFN-γ/IL4 ratio increased from 5.8 for the control group to 8.0 for the valine administration group. From these results, it was found that the Th2 predominance in inflammation reactions was corrected by valine administration.

**(Table 1)**

| | IFN-γ (pg/mL) | IL-4 (pg/mL) | IFN-γ/IL-4 |
|---|---|---|---|
| Control | 403.8 | 69.1 | 5.8 |
| Valine | 970.4 | 120.7 | 8.0 |

### (Example 8)

### <Effects of valine and ornithine administration on immune cell infiltration to lung tissue in ovalbumin-induced asthma model mice>

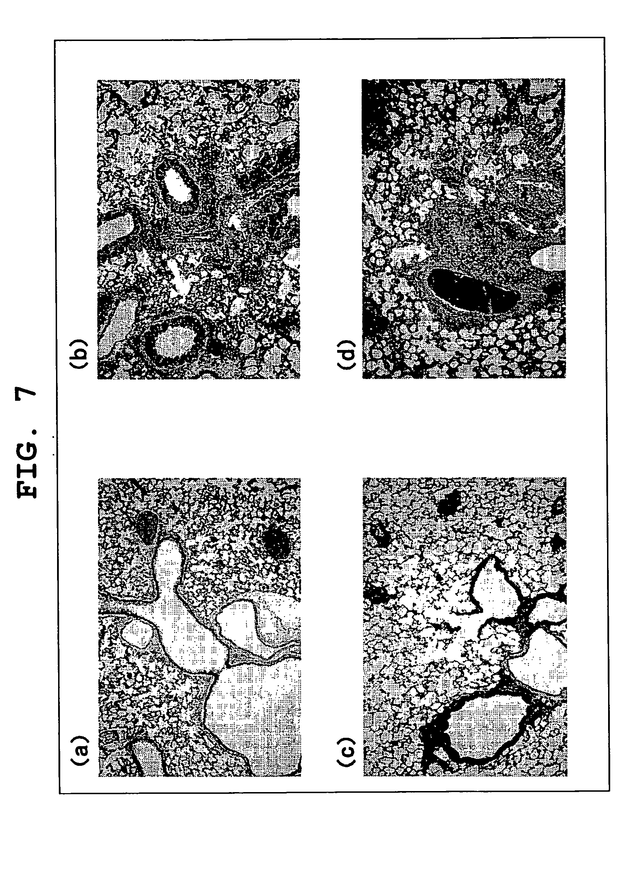

An ovalbumin-induced asthma model was prepared by a conventional method using Balb/c mice, and the drug effects of L-valine (free form) and ornithine hydrochloride were investigated. After elapse of 24 hours following a third antigen exposure, the lungs were extirpated from the mice and fixed in 10% formalin buffer solution, paraffin sections were prepared, and H/E staining was performed. After a 3 mM aqueous solution of valine and a 15 mM aqueous solution of ornithine hydrochloride were prepared, each was administered by the free water drinking method from day 0 to day 21. As a result, in the control group, bronchial stenosis that accompanied infiltration of Immune cells into lung tissue, hypertrophy of bronchial epithelial cells, appearance of mucosa-producing cells and the like occurred (see Figure 7(b)). On the other hand, in the valine administration group, all of the above-described symptoms were potently suppressed, and no bronchial stenosis was observed (see Figure 7(c)). From these results, an asthma suppressive effect of valine administration was confirmed also by the pathological analysis. On the other hand, in the ornithine administration group, no amelioration of the symptoms was observed (see Figure 7(d)). Note that Figure 7(a) is a lung tissue image of a normal mouse without asthma induction.

### (Example 9)

### <Effect of coadministration of valine and dexamethasone on ovalbumin-induced asthma model mice>

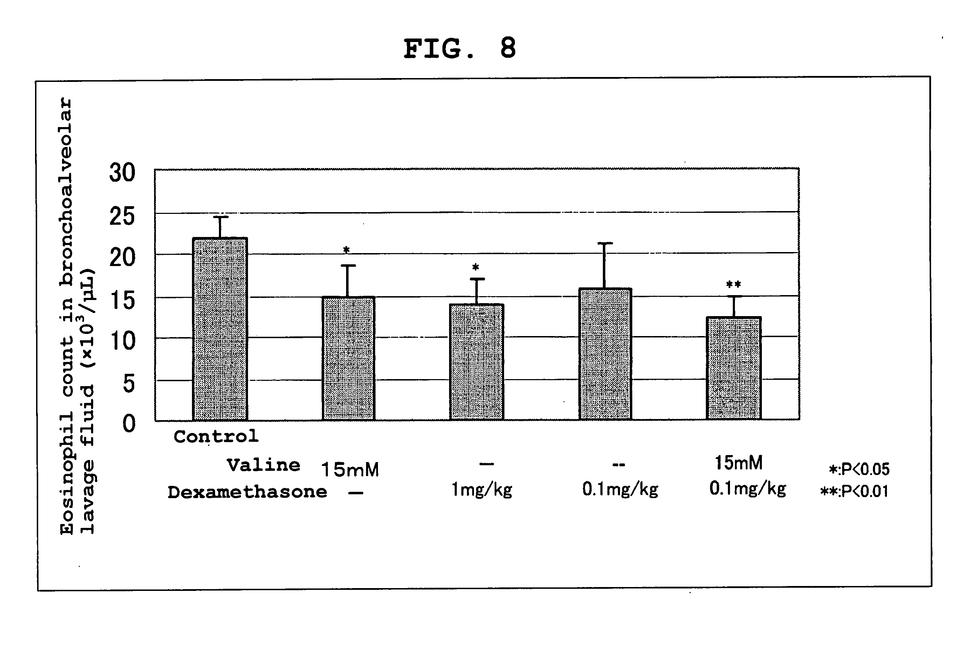

An ovalbumin-induced asthma model was prepared by a conventional method using Balb/c mice, and the effect of coadministration of L-valine (free form) and dexamethasone was investigated. L-valine (free form) began to be administered by the free water drinking method from day 0, and dexamethasone began to be administered once daily by intraperitoneal administration after a first suction exposure. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. After a 15 mM aqueous solution of valine was prepared, it was administered by the free water drinking method from day 0 to day 21, and 0.1 mg/kg or 1 mg/kg dexamethasone was intraperitoneally administered from day 12 to day 21. As a result, when L-valine and dexamethasone were coadministered, a more potent suppressive effect was observed compared to administration of dexamethasone alone in a 10-fold amount. From this result, it was found that the dose of dexamethasone could be reduced to one-tenth by coadministration with L-valine (see Figure 8). (Example 10)

### <Effect of administration of a mixture of valine and ornithine hydrochloride on ovalbumin-induced asthma model mice>

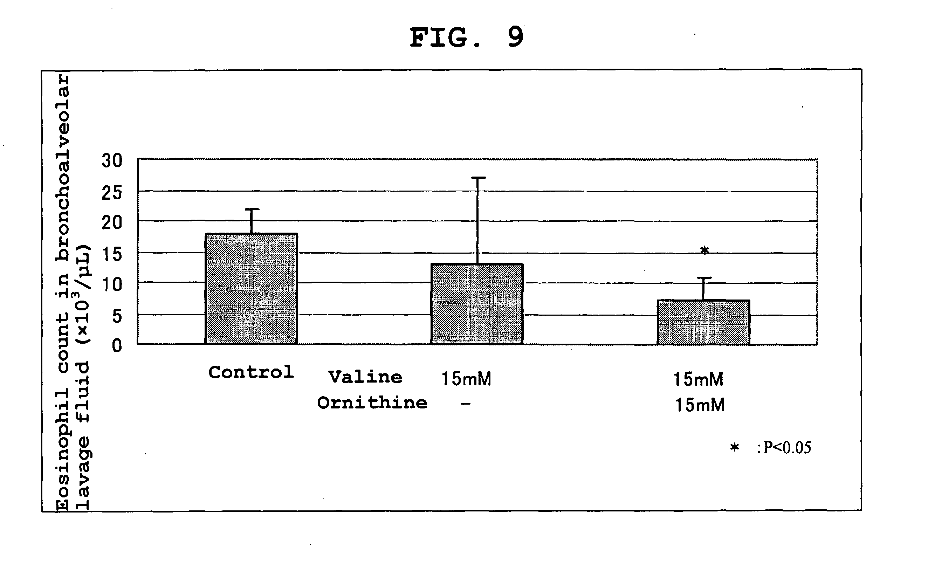

An ovalbumin-induced asthma model was prepared by a conventional method using Balb/c mice, a mixture of L-valine (free form) and ornithine hydrochloride (both at 15 mM) began to be administered by the free water drinking method after initiation of a first suction exposure (day 12). After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. Because the length of L-valine administration was insufficient, no significant effect of L-valine alone was observed in this experiment. However, when L-valine and ornithine hydrochloride were administered in combination, a significant suppressive effect was observed compared to the control group. With ornithine alone, the allergic symptoms in the asthma model mice were not ameliorated (see Example 8 and Figure 7), but it was found that by using L-valine and ornithine hydrochloride in combination, the allergic symptom ameliorating effect was synergistically enhanced (see Figure 9).

### (Example 11)

### <Changes in immune cell-produced cytokines by valine administration to ovalbumin-induced asthma model mice>

Ovalbumin-immunized Balb/c mice were given L-valine (free form), and changes in the cytokine productivity of isolated immune cells were examined, whereby the effect on the Th1/Th2 balance in a living organism's immune system was investigated. After a 3 mM aqueous solution of valine was prepared, it was administered by the free water drinking method from day 0 to day 11. 8 µg of ovalbumin and 2 mg of Alum, which is an adjuvant, per mouse were twice (day 0 and day 5) intraperitoneally injected. On day 11, macrophages were isolated from the lungs and cultured in the presence of 1 mg/mL ovalbumin overnight, after which they were further co-cultured with splenocytes isolated from an ovalbumin-immunized mouse for 48 hours. After the cultivation, cytokines in the culture supernatant were measured by an ELISA method. The measurement results are shown in Table 2. As a result, in the control group, the content of IFN-γ, which is a Th1 cytokine, was 147 pg/mL, and the content of IL-4, which is a Th2 cytokine, was 25.1 pg/mL; in the valine administration group, the contents of these cytokines were 341.1 pg/mL and 9.2 pg/mL, respectively. With valine administration, an increase in the Th1 cytokine content and a reduction in the Th2 cytokine content were observed. It is a well-known fact that in a living organism suffering a Th2 disease like asthma, Th2 becomes predominant in the immune system (see Holtzman et al, Am. J. Respir. Cell Mol. Biol. 14,316-318, 1996), and it was found that by valine administration, Th2 predominance was corrected.

**(Table 2)**

| APC types | IL-4 (pg/mL) | | IFN-γ (pg/mL) | |
|---|---|---|---|---|
| | Mean | SD | Mean | SD |
| Control | 25.1 | 4.7 | 147.0 | 19.7 |
| Valine | 9.2 | 1.5 | 341.1 | 20.8 |

### (Example 12)

### <Effect of administration of a mixture of valine and arginine on ovalbumin-induced asthma model mice>

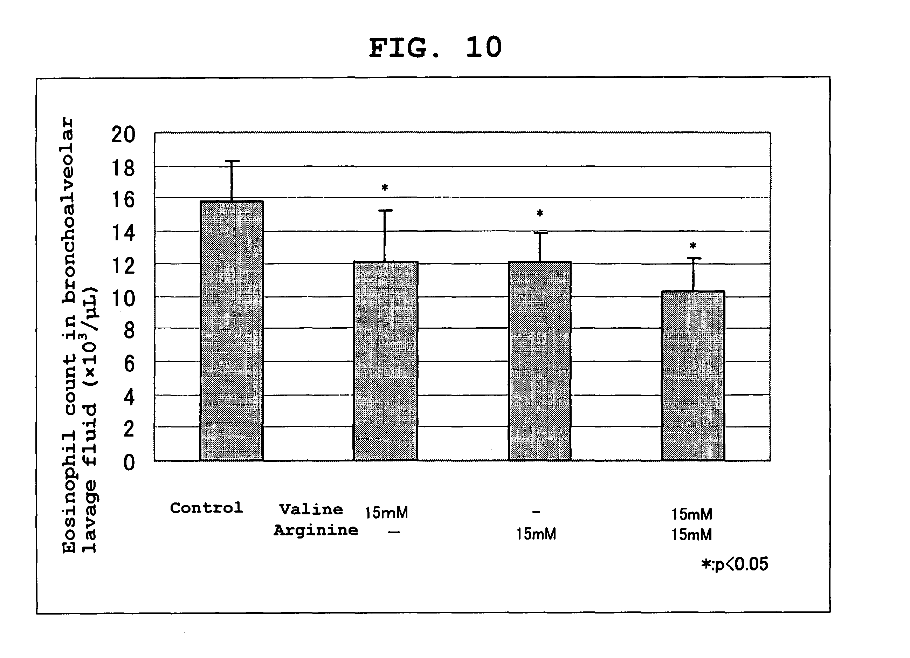

An ovalbumin-induced asthma model was prepared by a conventional method using Balb/c mice. After a 15 mM aqueous solution of L-valine (free form), a 15 mM aqueous solution of arginine, and a mixture of L-valine (free form) and arginine (both at 15 mM) were prepared, each aqueous solution was administered by the free water drinking method to the asthma model mice from day 0 to day 21. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. When L-valine and arginine were administered in combination, a significant suppressive effect was observed compared to the control group. Because a more potent suppressive effect was exhibited than when L-valine or arginine was administered alone, it was shown that by using L-valine and arginine in combination, the allergic symptom ameliorating effect was enhanced (see Figure 10).

### (Example 13)

### <Effect of valine administration by Oral administration method on ovalbumin-induced asthma model mice>

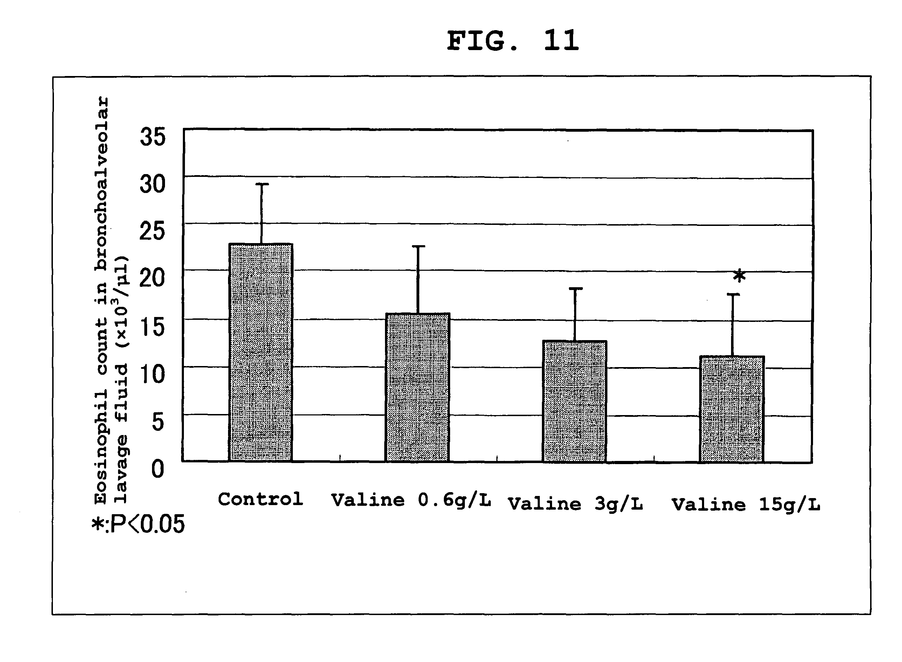

An ovalbumin-induced asthma model was prepared by a conventional method using Balb/c mice. A 0.6, 3, or 15 g/L aqueous solution of L-valine (free form) was administered by Oral administration method using a sonde at a dose of 100 (µL/time/animal twice a day for 7 days/week from day 0 to day 21. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. As a result, the eosinophil count in the BAL Fluid decreased with dependence on the dose of L-valine. It was found that L-valine had an allergic symptom ameliorating effect even when administered by Oral administration method twice a day (see Figure 11).

### (Example 14)

### <Effect of valine administration by inhalation on ovalbumin-induced asthma model mice>

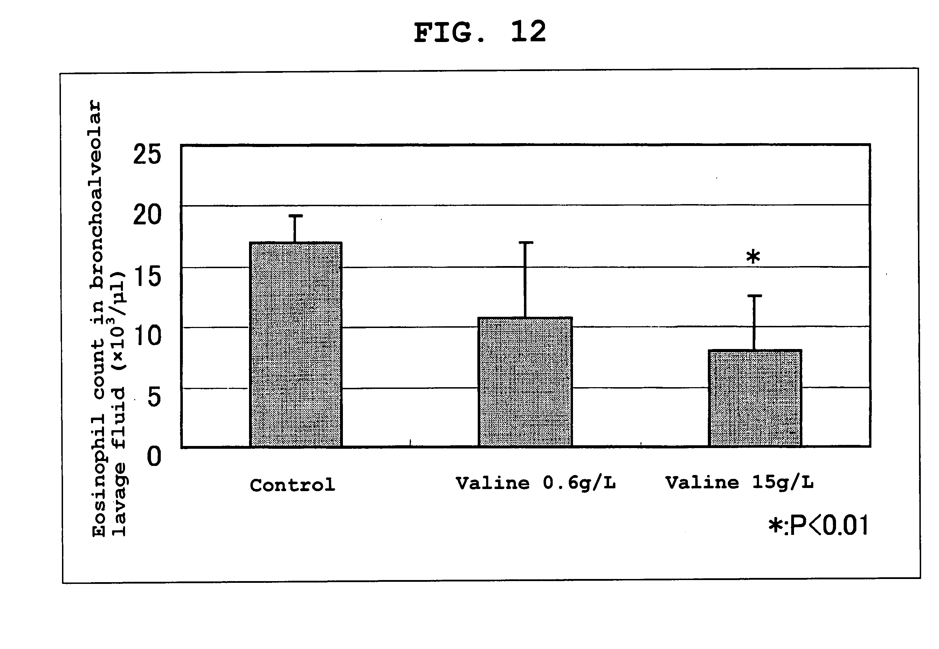

An ovalbumin-induced asthma model was prepared by a conventional method using Balb/c mice. A 0.6 or 15 g/L aqueous solution of L-valine (free form) was aerosolized using a nebulizer and administered by inhalation to asthma model mice once daily for 30 minutes for 5 days/week from day 11 to day 21. After elapse of 24 hours following a third antigen exposure, 7 mL of physiological saline was injected from the bronchia to achieve lung lavage, and the eosinophil count in the BAL Fluid was measured using an automated blood cell counter and evaluated as an index of localized lung inflammation. As a result, the eosinophil count in the BAL Fluid decreased with dependence on the dose of L-valine. It was found that L-valine had an allergic symptom ameliorating effect even when administered by inhalation (see Figure 12).

### (Example 15)

### <Anti-inflammatory effect of coadministration of valine and dexamethasone by inhalation on ovalbumin-induced pulmonary fibrosis model mice>

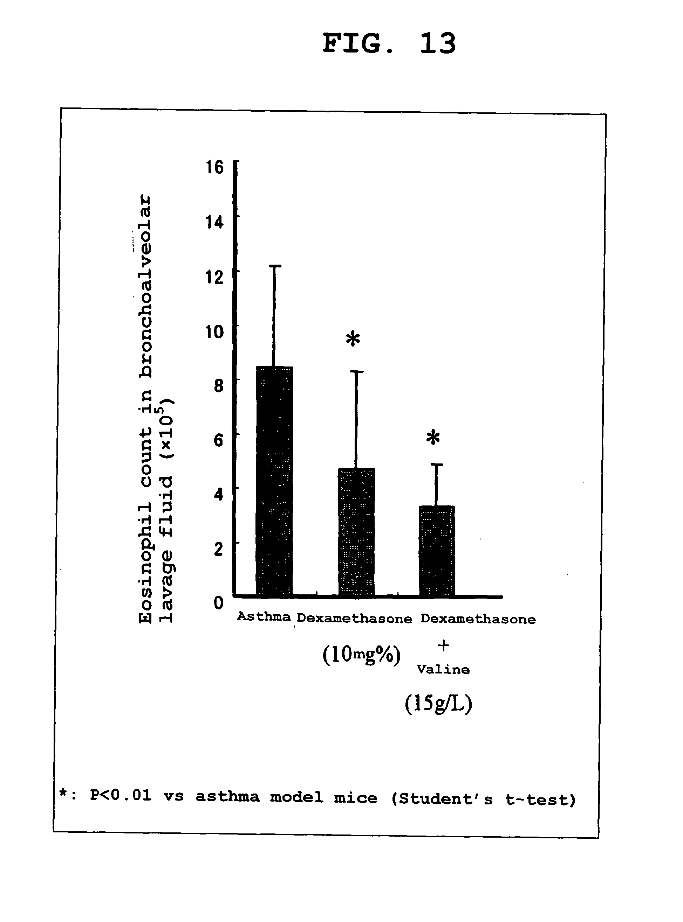

Ovalbumin-induced pulmonary fibrosis model mice were induced by a conventional method using Balb/c mice, and the lung function reduction suppressive effect of coadministration by inhalation of L-valine (free form) and dexamethasone was investigated. After 8 µg of ovalbumin and 2 mg of Alum, which is an adjuvant, per mouse were twice intraperitoneally injected on day 1 and day 11, 1.5% ovalbumin solution was aerosolized with a nebulizer and each mouse was subjected to suction exposure for 30 minutes for 3 weeks from day 21 to day 42. Valine (15 g/L) and dexamethasone (10 mg%) were aerosolized with a nebulizer and coadministered by inhalation (40 minutes per day) on consecutive days from day 21 to day 42. 24 hours after antigen exposure on day 42, 7 ml of physiological saline was injected from the bronchia to achieve lung lavage, and the eosinophil count in the BAL Fluid was measured using an automated blood cell counter and evaluated as an index of localized lung inflammation. As a result, in the group receiving dexamethasone and valine coadministered by inhalation, a statistically significant reduction was observed compared to the asthma induction group (no treatment), and a decreasing tendency was also observed compared to the dexamethasone-alone administration group. From this fact, it was shown that when valine was coadministered with dexamethasone by inhalation, localized inflammation in the lungs was more potently suppressed than when dexamethasone was administered alone (see Figure 13). Note that mg% indicates a weight per 100 cc of solvent (the same applies below).

### (Example 16)

### <Airway resistance ameliorating effect of coadministration of valine and dexamethasone by inhalation on ovalbumin-induced pulmonary fibrosis model mice>

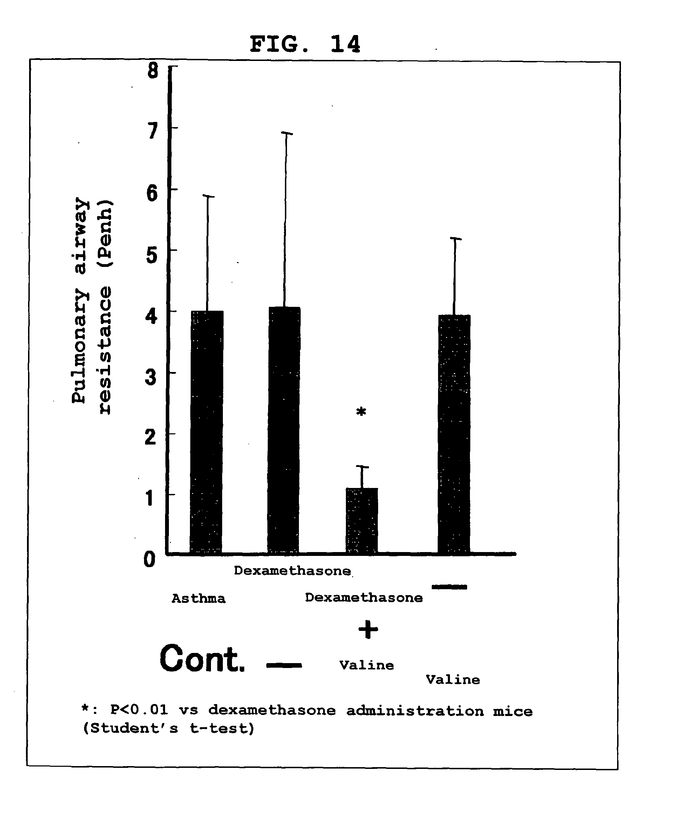

Ovalbumin-induced pulmonary fibrosis model mice were induced by a conventional method using Balb/c mice, and the lung function reduction suppressive effect of coadministration of L-valine (free form) and dexamethasone by inhalation was investigated. 24 hours after antigen exposure on day 42, the pulmonary fibrosis model mice were allowed to inhale methacholine, which is a bronchial constriction inducer, and airway resistance (Penh) was measured using a Buxco whole body plethysmography. Valine (15 g/L) and dexamethasone (10 mg%) were aerosolized with a nebulizer and coadministered by inhalation (40 minutes per day) on consecutive days from day 21 to day 42. As a result, in the asthma induction group (no treatment), the Penh value was 4.0, and in the dexamethasone-or valine-alone administration group, the Penh value was 4.1 and 4.0, respectively; in the group receiving dexamethasone and valine coadministered by inhalation, the Penh value was low at 1.1. From this fact, it was shown that when valine was coadministered with dexamethasone by inhalation, the lung function reduction was more potently suppressed than when dexamethasone was administered alone (see Figure 14).

### (Example 17)

### <Pulmonary fibrosis suppressive effect of coadministration of valine and dexamethasone by inhalation on ovalbumin-induced pulmonary fibrosis model mice>

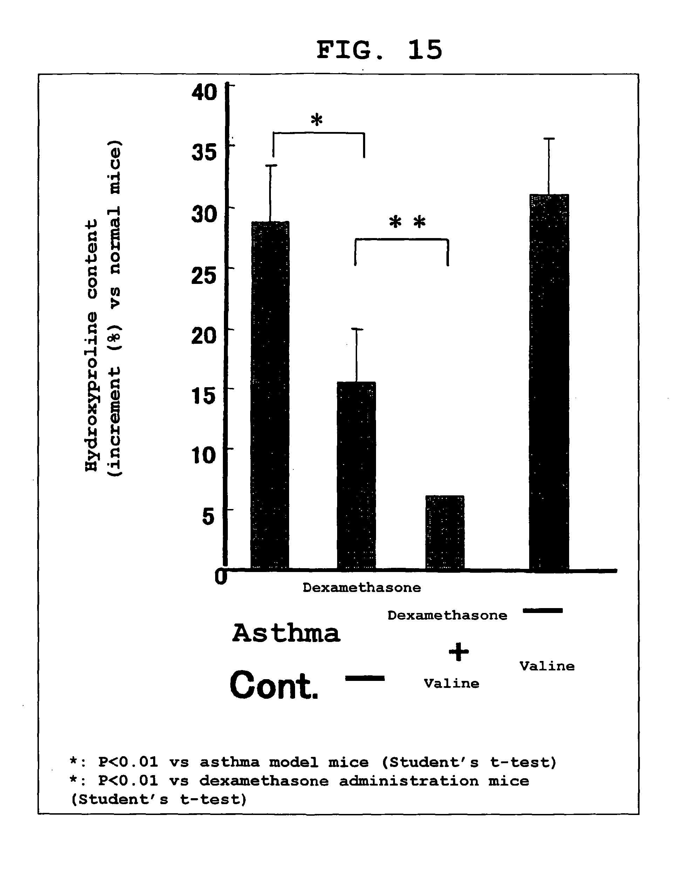

Ovalbumin-induced pulmonary fibrosis model mice were induced by a conventional method using Balb/c mice, and the pulmonary fibrosis suppressive effect of coadministration of L-valine (free form) and dexamethasone by inhalation was investigated. 24 hours after antigen exposure on day 42, the lungs were extirpated from the pulmonary fibrosis model mice, and the hydroxyproline content in lung tissue was measured as an index of the degree of fibrosis. Valine (15 g/L) and dexamethasone (10 mg%) were aerosolized with a nebulizer and coadministered by inhalation (40 minutes per day) on consecutive days from day 21 to day 42. The results are expressed as increments (%) of the hydroxyproline content in each test group compared to the hydroxyproline content in the lung tissues from normal mice. In the asthma induction group (no treatment), the hydroxyproline content increased by 28.7% compared to the lung tissue from the normal mice; in the dexamethasone- and valine-alone administration groups, the hydroxyproline content increased by 15.4% and 31%, respectively. On the other hand, in the valine and dexamethasone inhalation coadministration group, the hydroxyproline content increased by 6%. From this fact, it was shown that when valine was coadministered with dexamethasone by inhalation, pulmonary fibrosis was more potently suppressed than when dexamethasone was administered alone (see Figure 15).

### Industrial Applicability

According to the present invention, it is possible to provide a drug capable of preventing or treating an allergic disease caused by Th2 cell-polarized activation. Because the prophylactic/therapeutic agent of the present invention produces almost no side effects, it can be taken for a long time not only by adults but also by children and pregnant women; when the prophylactic/therapeutic agent of the present invention is used in combination with an anti-inflammatory drug such as a steroid drug or with a bronchodilator and the like, the dose of the drug can be reduced. Furthermore, according to the present invention, there is provided a food for the prevention/treatment of an allergic disease caused by functional accentuation of Th2 cells, that can be safely ingested routinely.

While some of the embodiments of the present invention have been described in detail in the above, it will, however, be evident for those of ordinary skill in the art that various modifications and changes may be made to the particular embodiments shown without substantially departing from the teaching and advantages of the present invention. Such modifications and changes are also encompassed in the spirit and scope of the present invention as set forth in the appended claims.

This application is based on a patent application No. 2004-319640 filed in Japan, the contents of which are incorporated in full herein by this reference.

## Claims

1. A prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient.

2. The prophylactic/therapeutic agent of claim 1, wherein the allergic disease is bronchial asthma.

3. The prophylactic/therapeutic agent of claim 1, wherein the allergic disease is pulmonary fibrosis that accompanies bronchial asthma.

4. The prophylactic/therapeutic agent of any one of claims 1 to 3, wherein the branched amino acid is selected from the group consisting of valine, leucine and isoleucine.

5. The prophylactic/therapeutic agent of any one of claims 1 to 4, wherein the keto acid is selected from the group consisting of 2-oxoisovaleric acid, 3-methyl-2-oxovaleric acid, 4-methyl-2-oxovaleric acid and glyoxylic acid.

6. The prophylactic/therapeutic agent of any one of claims 1 to 5, which further comprises ornithine or a salt thereof.

7. A combination drug comprising a combination of the prophylactic/therapeutic agent of any one of claims 1 to 6 and an anti-inflammatory drug.

8. The combination drug of claim 7, wherein the anti-inflammatory drug is a steroid drug.

9. A food for the prevention/treatment of an allergic disease caused by Th2 cell-polarized activation, which comprises at least one kind of compound selected from the group consisting of a branched amino acid, glycine, serine, arginine, a keto acid thereof and a salt thereof.

10. The food of claim 9, which is a food with health claims or a dietary supplement.

11. The food of claim 10, wherein the food with health claims is a food for specified health uses or a food with nutrient function claims.

12. The food of any one of claims 9 to 11, wherein the branched amino acid is selected from the group consisting of valine, leucine and isoleucine.

13. The food of any one of claims 9 to 12, wherein the keto acid is selected from the group consisting of 2-oxoisovaleric acid, 3-methyl-2-oxovaleric acid, 4-methyl-2-oxovaleric acid and glyoxylic acid.

14. The food of any one of claims 9 to 13, which further comprises ornithine or a salt thereof.

15. A commercial package comprising a composition comprising at least one kind selected from the group consisting of a branched amino acid, glycine, serine, arginine, a keto acid thereof and a salt thereof as an active ingredient, and a printed matter bearing the statement that the composition can be used, or should be used, for the prevention/treatment of an allergic disease caused by Th2 cell-polarized activation.

16. A commercial package comprising a combination drug comprising a composition comprising at least one kind selected from the group consisting of a branched amino acid, glycine, serine, arginine, a keto acid thereof and a salt thereof as an active ingredient and an anti-inflammatory drug, and a printed matter bearing the statement that the combination drug can be used, or should be used, for the prevention/treatment of an allergic disease caused by Th2 cell-polarized activation.

17. A commercial package comprising a food comprising at least one kind of compound selected from the group consisting of a branched amino acid, glycine, serine, arginine, a keto acid thereof and a salt thereof, and a printed matter bearing the statement that the food can be used, or should be used, for the prevention/treatment of an allergic disease caused by Th2 cell-polarized activation.

18. A use of a branched amino acid, glycine, serine, arginine, a keto acid thereof or a salt thereof for producing a prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient.

19. A use of at least one kind of compound selected from the group consisting of a branched amino acid, glycine, serine, arginine, a keto acid thereof and a salt thereof, and an anti-inflammatory drug, for producing a combination drug comprising a combination of a prophylactic/therapeutic agent for an allergic disease caused by Th2 cell-polarized activation, which comprises at least one kind selected from the group consisting of a branched amino acid, glycine, serine, arginine, a keto acid thereof and a salt thereof as an active ingredient, and an anti-inflammatory drug.

20. A use of a branched amino acid, glycine, serine, arginine, a keto acid thereof and a salt thereof for producing a food for the prevention/treatment of an allergic disease caused by Th2 cell-polarized activation, which comprises at least one kind of compound selected from the group consisting of a branched amino acid, glycine, serine, arginine, a keto acid thereof and a salt thereof.

21. The use of any one of claims 18 to 20, wherein the allergic disease is bronchial asthma.

22. The use of any one of claims 18 to 20, wherein the allergic disease is pulmonary fibrosis that accompanies bronchial asthma.

23. The use of any one of claims 18 to 22, wherein the branched amino acid is selected from the group consisting of valine, leucine and isoleucine.

24. The use of any one of claims 18 to 23, wherein the keto acid is selected from the group consisting of 2-oxoisovaleric acid, 3-methyl-2-oxovaleric acid, 4-methyl-2-oxovaleric acid and glyoxylic acid.

25. The use of any one of claims 18 to 24, which further comprises ornithine or a salt thereof.

26. A prophylactic/therapeutic method for an allergic disease caused by Th2 cell-polarized activation, which comprises administering an effective amount of at least one kind selected from the group consisting of a branched amino acid, glycine, serine, arginine, a keto acid thereof and a salt thereof to a subject of administration.

27. A prophylactic/therapeutic method for an allergic disease caused by Th2 cell-polarized activation, which comprises administering an effective amount of at least one kind selected from the group consisting of a branched amino acid, glycine, serine, arginine, a keto acid thereof and a salt thereof, and an anti-inflammatory drug, to an subject of administration.

28. A prophylactic/therapeutic method for an allergic disease caused by Th2 cell-polarized activation, which comprises ingesting a food comprising at least one kind of compound selected from the group consisting of a branched amino acid, glycine, serine, arginine, a keto acid thereof and a salt thereof.

29. The method of any one of claims 26 to 28, wherein the allergic disease is bronchial asthma.

30. The method of any one of claims 26 to 28, wherein the allergic disease is pulmonary fibrosis that accompanies bronchial asthma.

31. The method of any one of claims 26 to 30, wherein the branched amino acid is selected from the group consisting of valine, leucine and isoleucine.

32. The method of any one of claims 26 to 31, wherein the keto acid is selected from the group consisting of 2-oxoisovaleric acid, 3-methyl-2-oxovaleric acid, 4-methyl-2-oxovaleric acid and glyoxylic acid.

33. The method of any one of claims 26 to 32, which further comprises ornithine or a salt thereof.
